Europäisches Patentamt

European Patent Office

Office européen des brevets

(19)

(11) Publication number: 0 375 610
A2

(12) **EUROPEAN PATENT APPLICATION**

(21) Application number: 89810940.0

(22) Date of filing: 12.12.89

(51) Int. Cl.⁵: **C07H 15/14, C11D 1/66,
A61K 47/26**

(30) Priority: 19.12.88 US 286553
18.05.89 US 353586

(43) Date of publication of application:
27.06.90 Bulletin 90/26

(84) Designated Contracting States:
BE CH DE FR GB IT LI NL

(71) Applicant: CIBA-GEIGY AG
Klybeckstrasse 141
CH-4002 Basel(CH)

(72) Inventor: Falk, Robert A.
35 Glenside Drive
New City New York 10956(US)
Inventor: Clark, Kirtland P.
10 Allan Way
Bethel Connecticut 06801(US)
Inventor: Coughlin, Gregory R.
60 A Mosemann Ave.
Katonah New York 10536(US)

(54) **Perfluoroalkylthioglycosides.**

(57) Novel nonionic perfluoroalkylthioglycosides of the formula $R_f$-E-S-saccharide are described, wherein $R_f$ is a straight or branched chain perfluoroalkyl of 1 to 18 carbon atoms or said perfluoroalkyl substituted by perfluoroalkoxy of 2 to 6 carbon atoms, E is a connecting group, and the saccharide is a mono-, di-, or higher oligosaccharide comprising 1 to 30 units of 5, 6, or 7 carbon-membered sugars. They can be used as surfactants.

EP 0 375 610 A2

## PERFLUOROALKYLTHIOGLYCOSIDES

This invention relates to perfluoroalkylthioglycosides and their use as surfactants in biochemistry and in general surfactant applications.

Alkyl glycosides are well known and have long been used for biochemical research. However, they are expensive detergents and their use has been limited to small scale experiments.

Alkyl thioglycosides are disclosed in Japanese Patent Publication Sho 61/7288. These compounds possess much improved properties over the O-analogs and have many of the desirable properties usually required for solubilization, purification and reconstitution of membrane proteins. These properties include a high solubilizing power, non-denaturation of proteins, a high Critical Micelle Concentration (CMC), a nonionic nature, optical transparency, high chemical purity, high solubility in water, stability to acidic and enzymatic hydrolysis, inertness to protein assays, and ease of synthesis.

Some perfluoroalkyl glycosides have been reported, for example 1,1,1-trifluoroethyl glucoside in Biochem. 9, (140)2890-6(1970), and oligomeric glycosides derived from 1,1,11-trihydroperfluoroundecanol, in U.S. Patent No. 3,598,865. No perfluoroalkylthiosaccharides have previously been disclosed.

One aspect of this invention relates to novel perfluoroalkyl-thioglycosides.

These compounds have hight solubilizing power, electrical neutrality, and are considerably more stable than known alkyl perfluoroalkylglycosides. Further, they possess numerous other desirable properties; they are non-denaturing to proteins, they have a high CMC, they are nonionic in nature, they are optically transparent, they have high chemical purity, they have a high solubility in water, they are stable to acidic and enzymatic hydrolysis, they are inert to protein assays, and they are easily synthesized.

Another aspect of this invention relates to the use of such novel fluorochemical surfactants in artificial blood formulations since they are inherently compatible with the fluorochemicals used in such blood substitutes.

In addition, this invention provides a method to prepare stable fluorochemical emulsions, a method for the solubilization and reconstitution of proteins, and methods for enzyme inhibition.

The perfluoroalkylthioglycosides of this invention have the general formula

$R_f$-E-S-saccharide

where $R_f$ is a straight or branched chain perfluoroalkyl of 1 to 18 carbon atoms or said perfluoroalkyl substituted by perfluoroalkoxy of 2 to 6 carbon atoms,

E is a branched or straight chain alkylene of 1 to 10 carbon atoms, or said alkylene interrupted by one to three groups selected from the group consisting of -NR-, -O-, -S-, -SO$_2$-, -COO-, -OOC-, -CONR-, -NRCO-, -SO$_2$NR-, and NRSO$_2$-, or is terminated at the $R_f$ end with -CONR- or -SO$_2$NR-, where $R_f$ is attached to the carbon or sulfur atom, R is independently hydrogen, alkyl of 1 to 6 carbon atoms or hydroxyalkyl of 2 to 6 carbon atoms, and

saccharide is a mono-, di-, or higher oligosaccharide comprising 1-30 units of a 5,6, or 7 carbon-membered sugar. Preferably saccharide means glucose, fructose, mannose, galactose, talose, allose, altrose, idose, arabinose, xylose, lyxose, ribose, or mixture thereof.

Preferably the instant compounds of formula I are those where $R_f$ is a straight or branched chain perfluoroalkyl of 1 to 12 carbon atoms or perfluoroalkyl of 2 to 6 carbon atoms subsituted by perfluoroalkoxy of 2 to 6 carbon atoms. Perferably E in formula I is a straight or branched chain alkylene of 2 to 6 carbon atoms, -CONHCH$_2$CH$_2$-, -CH$_2$CH$_2$N(CH$_3$)CH$_2$CH$_2$-, -CH$_2$CH$_2$SO$_2$NHCH$_2$CH$_2$-, -CH$_2$CH$_2$OCH$_2$CH$_2$-, or SO$_2$NHCH$_2$CH$_2$-. R in formula I is preferably independently hydrogen, alkyl of 1 to 6 carbon atoms or hydroxyalkyl of 2 to 6 carbon atoms.

Saccharide is preferably a mono-, di- or higher oligosaccharide comprising 1 to 6, most preferably 1 to 3, units of a 5, 6, or 7 carbon-membered sugar or mixture thereof.

Most preferred are those compounds of formula I where $R_f$ is a straight or branched chain perfluoroalkyl of 6 to 12 carbon atoms. E is most preferably ethylene. Most preferably saccharide is comprised of 1 to 3 units of 6 carbon-membered sugars.

Particularly preferred are glucose, mannose or galactose, most particularly glucose.

A preferred group of compounds are those, wherein in formula I, $R_f$ is perfluoroalkyl of 6 to 12 carbon atoms, E is ethylene and saccharide is a mono-, di-, or higher oligosaccharide comprising 1 to 3 units of a 5, 6, or 7 carbon-membered sugar.

It is understood that the $R_f$ group usually represents a mixture of perfluoroalkyl moieties. When the $R_f$ group is identified as having a certain number of carbon atoms, said $R_f$ group also usually concomitantly contains a small fraction of perfluoroalkyl groups with a lower number of carbon atoms and a small fraction of perfluoroalkyl groups with a higher number of carbon atoms.

2

The $R_f$-thiosaccharides according to the invention can be obtained by the reaction of a perfluoroalkyl thiol of formula $R_f$-E-SH with an oligosaccharide or acylated (preferably acetylated) oligosaccharide containing from 1 to 30 units of 5, 6, or 7 carbon membered sugars.

In one preferred embodiment, the saccharide is glucose pentaacetate. In another preferred embodiment it is maltose octaacetate. These intermediates are commercially available in high purity; other intermediates are for example cellobiose octaacetate, galactose pentaacetate, pentoses, hexoses, heptoses, disaccharides, trisaccharides, tetrasaccharides, polysaccharides, and O-alkyl polysaccharides.

The synthesis of the thiosaccharides can be accomplished by the following methods, namely: (a) condensation between glycosyl halide esters and perfluoroalkylthiolates; (b) S-substitution of 1-thiosugars; (c) partial hydrolysis of aldose diperfluoroalkylacetals; (d) isomerization of other 1-thioglycosides; (e) direct, acid-catalyzed thiolysis of free sugars or glycosyl esters; and trans-thioglycosidation of O-alkyl polysaccharides with thiols.

Other procedures less commonly reported for synthesizing 1-thioglycosides may be used and involve (a) thiolysis of 3,4,6-tri-O-acetyl-1,2-anhydro-D-glucose; (b) pyrolysis of glycosyl xanthate esters; (c) photochemical additions of thiols to unsaturated compounds and (d) sulfur extrusions from glycosyl disulfides.

The thiolysis of aldoses, glycosides, and peracetylated sugars under acidic conditions is a generally applicable procedure which is an extension of the Helferich method for preparing acyl glycoside peracetates. The recent publications of Ferrier and Furneaux using $BF_3$ catalysis for thiolysis of sugar esters (1976-1980) have proven to be the method of choice.

The preferred synthetic method requires the use of boron trifluoride catalysis of a homogeneous reaction of the subject glycoside peresters and a perfluoroalkyl thiol in chloroform. The reaction proceeds more readily with trans-peresters but also with the cis-isomers. The reaction uses near equimolar proportions of thiols and proceeds smoothly at 20-65°C in chloroform, requiring between 1 and 50 hours, and giving moderately high yields of product. Other non-$BF_3$ reactive solvents can be used including carbon tetrachloride, methylene chloride, ethers, and the like.

The perester product is isolated by evaporation, chromatography, or crystallization. The perfluoroalkylthiosaccaride is obtained by deacetylation. A mixture of methanol, triethylamine and water may be used for deacetylation and requires from 1-3 days at room temperature. The product is separated from the acetate counterion by ion-exchange chromatography using a basic (OH) ion exchange resin, followed by freeze drying to isolate the pure thiosaccharide. For isolation procedures, see for example Koeltzow and Urfer in J. Am. Oil Chem. Soc. 61 (10), 1651-5 (1984).

Alternately, perfluoroalkyl thiols may be reacted by trans-thioglycosidation, similarly to the methods described for alcohols described in U.S. Patents Nos. 3,547,828, 4,510,306, 4,663,444. These documents generally disclose trans-glycosidation procedures which are economically practical.

Alternate reaction schemes based on Fischer type acetalizations, Koenigs Knorr, enzymatic, and alkali catalyzed thioether formation can be used. Further 1-S-substituted perfluoroalkyl-thiosaccharides may be obtained by using the methods taught in U.S. 4,663,444 (1987).

Perfluoroalkyl thiols useful herein are well documented in the prior art. For example, thiols of the formula $R_f$-E-SH have been described in U.S. Patents Nos. 3,655,732 and 4,584,143.

Particularly preferred herein are the thiols of formula
$R_f CH_2 CH_2 SH$
where $R_f$ is perfluoroalkyl of 6 to 12 carbon atoms. These $R_f$-thiols can be prepared from $R_f CH_2 CH_2 I$ and thiourea in very high yield.

The resultant perfluoroalkylthiosaccharides, particularly when prepared from pure, homogeneous saccharides, can be used to prepare fluorinated emulsions and microemulsions of fluorochemicals as synthetic blood substitutes.

Accordingly, the instant invention also pertains to a stable, aqueous emulsion, suitable for use as a synthetic blood substitute, which comprises

(a) a fluorochemical oxygen carrier, and

(b) an effective amount of a compound of the formula $R_f$-E-S-saccharide described above. Preferably such amounts of component (b) are in the range of 0.01 to 2.00 % by weight of the total emulsion.

It is expected that such emulsions will be useful as injectable $O_2$-carriers. The necessary criteria for such emulsions include solubilization of fluorochemical oxygen carriers, stability, nonionic character, chemical inertness, biological acceptance, non-hemolytic behaviour, and industrial feasibility.

The perfluoroalkylthiosaccharides according to the present invention exhibit high surface activity in aqueous solutions. Depending on the choice of the perfluoroalkyl and saccharide substituents, dilute solutions may exhibit surface tensions below 17 dynes/cm. at 0.01 % by weight of perfluoroalkyl-

3

thiosacchaaride added.

Accordingly, the present invention encompasses a method of decreasing the surface tension of an aqueous solution comprising the addition of an effective amount of a perfluoroalkylthioglycoside of the present invention, or a mixture thereof; preferably about 0.0001 to about 2.0 wt. percent is added.

The invention is illustrated by the following examples:

Example 1: a) 1,1,2,2-Tetrahydroperfluoro-octane-b-D-thioglucopyranoside tetraacetate

Penta-O-acetyl b-D-glucopyranose (30.0 g, 76.9 mmol) and 1,1,2,2-tetrahydroperfluoro-octanethiol (32.1 g, 84.6 mmol) are dissolved in 90 ml of chloroform followed by the addition of a catalytic amount of boron trifluoride etherate (1.5 g, 22.1 mmol). The mixture is heated under nitrogen reflux for 2 hours and then extracted twice with saturated sodium bicarbonate solution and twice with distilled water. The chloroform is then removed under vacuum to yield a clear syrup which solidifies into a white solid (73 % by GLC). Recrystallization from hexane/acetone (12:1) yields a white crystalline solid of m.p. 86-87$^\circ$ C (100 % by GLC).

b) 1,1,2,2-Tetrahydroperfluoro-octane-b-D-thioglucopyranose

The tetraacetate from the Example 1a is dissolved in a solution (300 ml) consisting of methanol, triethylamine and water (2:2:1) and stirred at room temperature for 15 hours until deacetylation is complete. The solution is then passed through a column packed with Amberlite IRA-400 (OH) ion-exchange resin. The product is eluted with methanol and isolated by removing the methanol under vacuum followed by freeze-drying to yield a white crystalline solid of m.p. 218$^\circ$ C (94 % by GLC).

NMR shows proton resonances at 2.59 ppm, 2 protons, $CF_2$-$\underline{CH_2}$; 2.93 ppm, 2 protons, $\underline{CH_2}$-S; 3.28 ppm, 4 protons, HO-C-$\underline{H}$; 3.64 ppm and 3.85 ppm, 1 proton each, $\overline{CH_2}$-OH; 4.42 ppm, 1 proton, $\overline{S-C-H}$.

| Surface Properties in Distilled Water (Dynes/cm) | |
| --- | --- |
| Concentration (% by weight) | Surface Tension |
| 0.01 | 16.9 |
| 0.001 | 36.2 |
| 0.0001 | 60.7 |

Example 2: a) 1,1,2,2-Tetrahydroperfluorooctane-b-D-thio Glucopyranoside Octaacetate

Octa-O-acetyl b-D-maltopyranose (20.0 g, 29.5 mmol) and 1,1,2,2-tetrahydroperfluorooctanethiol (12.3 g, 32.4 mmol) are dissolved in 70 ml of chloroform followed by the addition of a catalytic amount of boron trifluoride etherate (0.5 g, 7.3 mmol). The mixture is heated under nitrogen reflux for 2.5 hours and then extracted twice with saturated sodium bicarbonate solution and twice with distilled water. The chloroform is then removed under vacuum to yield a deep orange syrup. This syrup is dissolved in heptane/acetone (9:1), placed on a flash chromatography column packed with silica gel and eluted with hexane/acetone (9:1). Fractions are taken, followed by glc, and the solvent is removed from selective fractions under vacuum to yield a white crystalline solid of m.p. 105-107$^\circ$ C.

b) 1,1,2,2-Tetrahydroperfluoro-octane-b-D-thiomaltopyranose

The material from Example 2a is dissolved in a deacetylating solution (400 ml) consisting of methanol, triethylamine, and water (2:2:1). The solution is stirred at room temperature for 15 hours to complete the deacetylation. The solution is then placed on a column packed with Amberlite IRA-400 (OH) ion-exchange resin. The product is diluted with methanol and isolated by removing the methanol under vacuum, followed by freeze drying, to yield a white crystalline solid of m.p. 279° C (dec.).

| Surface Properties in distilled water: | | |
|---|---|---|
| Concentration (% by weight) | Surface Tension | Interfacial Tension |
| | dynes/cm | dynes/cm |
| 0.10 | 22.2 | 7.7 |
| 0.01 | 24.4 | 11.9 |
| 0.001 | 45.2 | 28.1 |

Examples 5-16:

Using the methods described and by techniques similar to Examples 1 and 2, thiosaccharides can be prepared from selected fluorochemical precursors and saccharide intermediates as shown in the following table

| Fluorochemical Precursor Intermediate | Saccharide |
|---|---|
| 5 $CF_3CF_2CH_2SH$ | glucose pentaacetate |
| 6 $C_6F_{13}(CH_2)_4SH$ | methyl glycoside |
| 7 $C_8F_{17}CH_2CH_2CH_2SH$ | maltose octaacetate |
| 8 $C_8F_{17}CH_2CH_2N(CH_3)CH_2CH_2CH_2SH$ | glucose pentaacetate |
| 9 $C_8F_{17}SO_2NHCH_2CH_2SH$ | galactose pentaacetate |
| 10 $C_8F_{17}CH_2CH_2I$ | 2,3,4-tri-O-acetyl-1-thio-L-fucopyranose |
| 11 $C_7F_{15}CONHCH_2CH_2SH$ | cellobiose octaacetate |
| 12 $CF_3CF_2CH_2CH_2SH$ | butyl maltooligosaccharide |
| 13 $C_8F_{17}CH_2CH_2SH$ | glucose |
| 14 $C_8F_{17}CH_2CH_2SO_2NHCH_2CH_2SH$ | glucose pentaacetate |
| 15 $C_6F_{13}CH_2CH_2I$ | 1-thio-b-D-mannose |
| 16 $C_8F_{17}CH_2CH_2SH$ | starch |

**Claims**

1. A compound of the formula I
$R_f$-E-S-saccharide     (I),
wherein
$R_f$ is a straight or branched chain perfluoroalkyl of 1 to 18 carbon atoms or said perfluoroalkyl substituted by perfluoroalkoxy of 2 to 6 carbon atoms,
E is branched or straight chain alkylene of 1 to 10 carbon atoms, or said alkylene interrupted by one to three groups selected from the group consisting of -NR-, -O-, -S-, -SO$_2$-, -COO-, -OOC-, -CONR-, -NRCO-, -SO$_2$NR-, and NRSO$_2$-, or is terminated at the $R_f$ end with -CONR- or -SO$_2$NR-, where $R_f$ is attached to the carbon or sulfur atom, R is independently hydrogen, alkyl of 1 to 6 carbon atoms or hydroxyalkyl of 2 to 6 carbon atoms, and
saccharide is a mono-, di-, or higher oligosaccharide comprising 1-30 units of a 5, 6, or 7 carbon-membered sugar or a mixture thereof.
2. A compound according to Claim 1 wherein $R_f$ is a straight or branched chain perfluoroalkyl of 1 to 12 carbon atoms or perfluoroalkyl of 2 to 6 carbon atoms substituted by perfluoroalkoxy of 2 to 6 carbon atoms.

3. A compound according to Claim 1 wherein said $R_f$ is a straight or branched chain perfluoroalkyl of 6 to 12 carbon atoms.

4. A compound according to Claim 1 wherein E is a straight or branched chain alkylene of 2 to 6 carbon atoms, $-CONHCH_2CH_2-$, $-CH_2CH_2N(CH_3)CH_2CH_2-$, $-CH_2CH_2SO_2NHCH_2CH_2-$, $-CH_2CH_2OCH_2CH_2-$, or $SO_2NHCH_2CH_2-$.

5. A compound according to Claim 1 wherein said E is a branched or straight chain alkylene of 2 to 6 carbon atoms.

6. A compound according to Claim 1 wherein said E is ethylene.

7. A compound according to Claim 1 wherein said saccharide is a mono-, di-, or higher oligosaccharide comprising 1 to 6 units of a 5, 6, or 7 carbon-membered sugar or mixture thereof.

8. A compound according to Claim 1 wherein said saccharide is a mono-, di-, or higher oligosaccharide comprising 1 to 3 units of a 5, 6, or 7 carbon-membered sugar or mixture thereof.

9. A compound according to Claim 1 wherein said sugar is a 6 carbon-membered sugar, comprising 1 to 3 units.

10. A compound according to Claim 1 wherein said sugar is selected from the group consisting of glucose, fructose, mannose, galactose, talose, allose, altrose, idose, arabinose, xylose, lyxose, ribose and mixtures thereof.

11. A compound according to Claim 11 wherein said sugar is selected from the group consisting of glucose, mannose, galactose and mixtures thereof.

12. A compound according to Claim 12 wherein said sugar is glucose.

13. A compound according to Claim 1 wherein $R_f$ is perfluoroalkyl of 6 to 12 carbon atoms, E is ethylene and saccharide is a mono-, di-, or higher oligosaccharide comprising 1 to 3 units of a 5, 6, or 7 carbon-membered sugar.

14. A process for the production of a compound of formula I according to claim 1 or mixtures thereof, which comprises reacting under acidic conditions a compound of formula $R_f$-E-SH with an oligosaccharide or acylated oligosaccharide containing from 1 to 30 units of 5, 6 or 7 carbon membered sugar, whereby $R_f$ and E have the meanings as defined in claim 1, and deacylating the reaction product.

15. A process according to claim 14, wherein the saccharide is acetylated.

16. A stable, aqueous emulsion, suitable for use as a synthetic blood substitute, which comprises
    (a) a fluorochemical oxygen carrier, and
    (b) an effective amount of a compound according to Claim 1.

17. A method of decreasing the surface tension of an aqueous solution comprising adding an effective amount of a compound or mixture of compounds according to Claim 1 to said aqueous solution.

18. A method according to Claim 17 wherein about 0.0001 to about 2.0 wt. % of a compound or mixture of compounds according to Claim 1 is added.